# EUROPEAN PATENT APPLICATION

(11) **EP 2 039 752 A1**
(43) Date of publication of application: **25.03.2009**
(21) Application number: 07116873.6
(22) Date of filing: 20.09.2007
(51) Int. Cl.: C12M 1/00

(54) **Incubator humidity control**

(71) Applicant: The Automation Partnership (Cambridge) Limited, Royston, Hertfordshire SG8 5WY (GB)
(72) Inventor: Grant, Paul Simon, Cambridge, Cambridgeshire CB4 3AG (GB)
(74) Representative: Brunner, Michael John

(57) **Abstract**

An incubator 1 for cell culture, or biological or chemical samples, the incubator having disposed therein a humidity sensor 28 and a humidifier 2 having an atomiser including a spray nozzle 21. A compressed air supply line 25 and a liquid supply line 26 are connected to the spray nozzle. A control system 30 allows the required humidity level to be set, and is connected to the humidity sensor 28 to receive a signal indicative of the humidity within the incubator, and is also connected to the humidifier 2 to operate the humidifier to maintain the humidity within the incubator at the set level.

## Description

The present invention relates to incubators for cell culture, or biological or chemical samples, for example of the type shown in EP-A-0569214 or EP-A-1657552.

More particularly, the invention is concerned with maintaining a desired level of humidity in such incubators.

It is well known that, in order to allow certain cell culture, or biological or chemical processes to function satisfactorily the samples held within vessels in such an incubator need to be maintained at both a desired temperature or range of temperatures and a suitable humidity or range of humidity. In particular, when using SBS Format or similar microtitre plates with loose fitting lids, because greater evaporation otherwise occurs at the wells adjacent the edges of the plates, maintaining a relatively high humidity helps to avoid lack of uniformity in evaporation.

While it has been proposed to provide incubators with humidifiers using either heated water sources or with ultrasonic atomisers, it is desirable to avoid adding heat through the humidifier because this will then create undesirable hot spots within the incubator. Ultrasonic units in particular, provide undesirable heat sources. A further problem with prior humidifying systems is that from normal lab conditions, conventional humidity devices may take an hour or more to restore high humidity (>90%), which is undesirable if there is continual access.

According to the present invention therefore there is provided an incubator for cell culture, or biological or chemical samples, the incubator having disposed therein:
a humidity sensor;
a humidifier having an atomiser including a spray nozzle, and a compressed air supply line and a liquid supply line to the spray nozzle; and
a control system in which the required humidity level can be set, connected to the humidity sensor to receive a signal indicative of the humidity within the incubator, and connected to the humidifier to operate the humidifier to maintain the humidity within the incubator at the set level.

Preferably, the humidifier includes a venturi.

Suitable humidifiers are manufactured by Delavan® and utilise compressed air to draw water into the nozzle over the venturi and to atomise it.

Preferably, the humidity sensor includes a PID controller and this is connected to a compressed air solenoid valve to determine the amount of compressed air and hence atomised water delivered into the incubator.

The system of the invention also provides a very fast recovery speed after humidity has dropped when the door has been opened by an operator or by the robotics outside the incubator.

If desired, the compressed air may be filtered, for example using a standard SMC filter to filter out particles such as undesirable bacteria, having a diameter greater than 0.1µm. The liquid (water) supply to the humidifier may also include a solenoid valve, depending on the head of water in the supply and may include suitable filters also.

One example of an incubator according to the present invention will now be described with reference to the accompanying drawings in which:
Figures 1 and 2 show a typical incubator with plural supports for multiple racks of sample flasks;
Figure 3 illustrates the components of a humidifying system associated with the incubator; and
Figure 4 illustrates the humidifier in more detail.

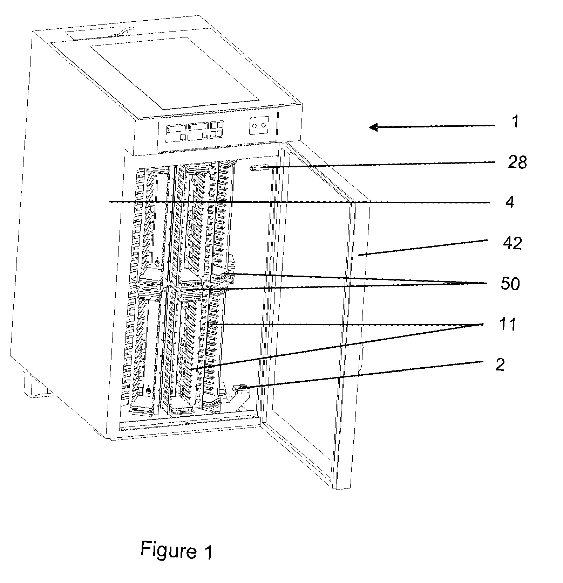

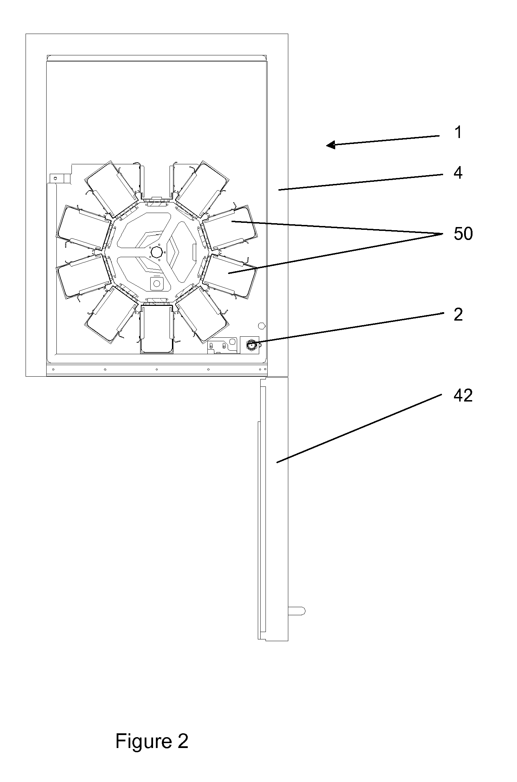

Figure 1A is a perspective view of an incubator and Figure 2 is a plan view of the incubator. The incubator 1 shown in the drawing includes a housing 4 in order to enable a controlled environment to be maintained and adjusted. The incubator housing 4 may include (not shown) a slidable access panel or door 41 through which a transporter in the form of a robotic arm with a gripper unit may be extended to load/unload culture vessels such as SBS format microtitre plates 50. The housing has a glass panelled operator access door 42.

The incubator 1 includes a plurality of microtitre plate 'hotels' 11 which are disposed so as to be rotatable for access, about a vertical axis generally centrally disposed within the incubator, the hotels 11 support the plates 50 in columns around the common axis to provide what is, effectively a carousel.

As the remaining components of the incubator shown in Figures 1 and 2 are not related to the present invention they will not be further described.

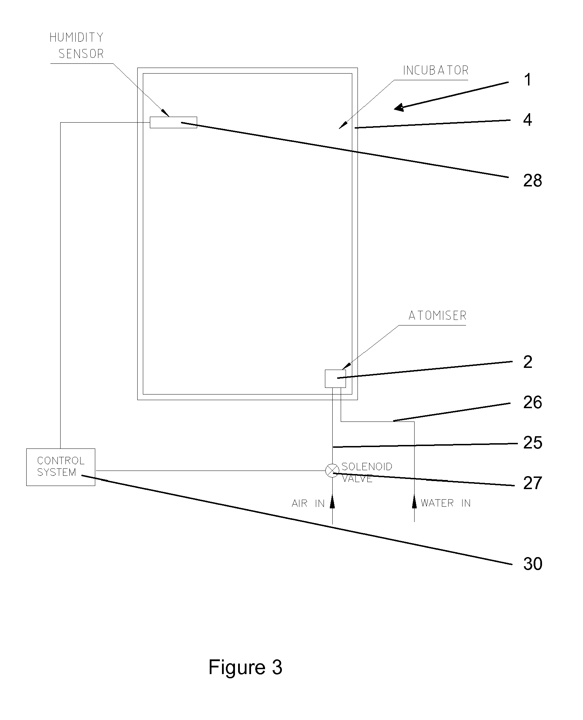

As can be seen in Figures 1 to 3, at the bottom of the incubator 1, there is positioned a humidifier 2 which includes a spray nozzle 21, a compressed air supply line connector 23 connecting a compressed air line 25, and a liquid supply line connector 22 connecting a water feed line 26 to the spray nozzle. The humidifier is mounted to the housing 4 via a bracket 24. An externally positioned control system 30 (see Figure 3) allows the required humidity level to be set by controlling a solenoid valve 27 in the compressed air line 25 which feeds the humidifier 2. The control system 30 is also connected to a humidity sensor 28, positioned inside and near the top of the incubator housing 4, in order to receive a signal indicating the humidity within the incubator 1. The control system 30 is connected to operate the humidifier to maintain the humidity within the incubator at a level set within the control system.

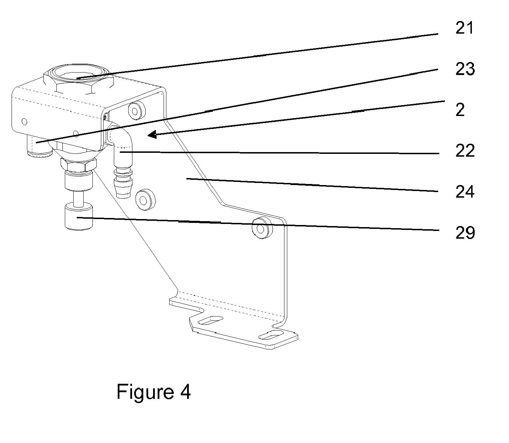

The humidifier shown in the drawings is a Delavan® AL air atomising series spray nozzle which uses the energy of the pressurised (compressed) air to atomise the water and this allows the water to be fed under lower pressure and still achieve fine atomisation. It includes an adjusting valve, the control knob 29 of which is seen in Figure 4

The combination of a humidifier 2 of this type with an appropriate humidity sensor 28 and control system 30 allows a simple and cost effective means of producing the desired humidity environment in the incubator 1 without introduction of any significant heat.

The system of the example can achieve 95%RH in a matter of a few minutes.

## Claims

1. An incubator for cell culture, or biological or chemical samples, the incubator having disposed therein:
a humidity sensor;
a humidifier having an atomiser including a spray nozzle, and a compressed air supply line and a liquid supply line to the spray nozzle; and
a control system in which the required humidity level can be set, connected to the humidity sensor to receive a signal indicative of the humidity within the incubator, and connected to the humidifier to operate the humidifier to maintain the humidity within the incubator at the set level.

2. An incubator according to claim 1, wherein the incubator has a venturi atomiser.

3. An incubator according to claim 1 or claim 2 further including a filter in the compressed air supply line.

4. An incubator according to any of claims 1 to 3, wherein the liquid supply line includes a filter disposed therein.
